(19) Europäisches Patentamt / European Patent Office / Office européen des brevets

(11) **EP 2 300 857 B1**

(12) **EUROPEAN PATENT SPECIFICATION**

(45) Date of publication and mention
of the grant of the patent:
**10.09.2014 Bulletin 2014/37**

(21) Application number: **09786509.1**

(22) Date of filing: **03.07.2009**

(51) Int Cl.:
*A61B 1/00* *(2006.01)*    *G02B 23/24* *(2006.01)*
*G02B 3/14* *(2006.01)*

(86) International application number:
**PCT/IB2009/052900**

(87) International publication number:
**WO 2010/004493 (14.01.2010 Gazette 2010/02)**

(54) **AN OPTICAL IMAGE PROBE**

OPTISCHE BILDSONDE

SONDE D'IMAGE OPTIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **10.07.2008 EP 08160097**

(43) Date of publication of application:
**30.03.2011 Bulletin 2011/13**

(73) Proprietor: **Koninklijke Philips N.V.
5656 AE Eindhoven (NL)**

(72) Inventors:
• **KUIPER, Stein
  NL-5656 AE Eindhoven (NL)**
• **HENDRIKS, Bernardus, H., W.
  NL-5656 AE Eindhoven (NL)**
• **MIHAJLOVIC, Nenad
  NL-5656 AE Eindhoven (NL)**

(74) Representative: **Kroeze, Johannes Antonius
Philips
Intellectual Property & Standards
P.O. Box 220
5600 AE Eindhoven (NL)**

(56) References cited:
WO-A1-2004/051323    FR-A1- 2 876 267
US-A- 4 930 861    US-A1- 2004 218 283
US-A1- 2007 156 021    US-B1- 6 476 979

**Description**

FIELD OF THE INVENTION

The present invention relates to an optical image probe, the probe is particularly suited for miniature application e.g. in-vivo. The invention also relates to a corresponding imaging system with the said optical image probe and a corresponding method for imaging.

BACKGROUND OF THE INVENTION

For correct diagnosis of various diseases biopsies are often taken. This can either be via a lumen of an endoscope or via needle biopsies. In order to find the correct position to take the biopsy, various imaging modalities are used such as X-ray, MRI and ultrasound. In case of e.g. prostate cancer in most cases the biopsy is guided by ultrasound. Although helpful, these methods of guidance are far from optimal. The resolution is limited and, furthermore, these imaging modalities can in most cases not discriminate between benign and malignant tissue. As a result a physician does not know for certain that from the correct part of the tissue a biopsy is taken. Thus, the physician takes almost blind biopsies and even if after inspection of the tissue no cancer cells are detected, one does not know for certain that simply the right spot to take the biopsy was missed.

In order to improve the biopsy procedure direct inspection of the biopsy position prior of taken the biopsy is required. A way to achieve this is by microscopic inspection at this position e.g. by an endoscopic inspection.

Considering the need to perform comprehensive and fast imaging by endoscopic inspection it is important for many applications to have the possibility of zooming. This is however difficult with mechanical means around the lenses due to the quite limited space. A zoom lens based on variable-focus fluid lenses has the advantage of no additional space consuming mechanical parts around the lenses. A switchable lens can be made according to the principles described in US patent 7,126,903 B2 (Variable Focus Lens). In this reference, it is described how a variable-focus lens can be made with the smallest possible outer diameter. This lens has the drawback that only a certain zoom factor can be reached due to the limited diopter change that can be reached by the fluid lens.

Document US 2007/0156021 discloses an endoscope having a fluid lens. Document US 4,930,861 discloses an electronic camera for endoscopes.

Hence, an improved optical image probe would be advantageous, and in particular a more efficient and/or reliable probe would be advantageous.

SUMMARY OF THE INVENTION

Accordingly, the invention preferably seeks to mitigate, alleviate or eliminate one or more of the above mentioned disadvantages singly or in any combination. In particular, it may be seen as an object of the present invention to provide an optical image probe that solves the above mentioned problems of the prior art with obtaining a relatively high zoom factor in a compact way.

This object and several other objects are obtained in a first aspect of the invention by providing an optical image probe comprising:

- a housing,
- a fluid lens positioned at an end portion of the housing, the fluid lens having a changeable optical power, and
- an image collector positioned within the housing, the image collector being arranged in an optical path of the fluid lens, the image collector being displaceable along the said optical path by an actuator.

The invention is particularly, but not exclusively, advantageous for obtaining a compact optical image probe and which simultaneously has a high zoom factor. Due the possible displacement of the image collector and the changeable optical power of the fluid lens, and cooperation between these two elements, it is possible to obtain a compact endoscope with a wide dynamic range of zoom factor with satisfactory focusing properties.

In order to be able to zoom one may thus make use of a fluid lens to change the effective focal length of the lens system of the probe (i.e. to zoom) and to use the displacement of the image collector to bring the lens system in focus. This may be performed simultaneously, consecutively (in both orders) or iteratively, or any combinations thereof. This way of zooming combines the best of both worlds: the fluid lens need only to switch over a small range, the large displacement can be achieved by an actuator mechanically connected to the image collector, and this actuator may therefore take relatively less lateral space than when it would have been designed immediately next to or around the lens elements (which would increase the endoscope diameter).

In general the present invention is implemented with an image collector which is diplaceably arranged on the optical path from the fluid lens, but it is to be understood for the skilled reader that the image collector, and the displacement thereof, may specifically be one or more fibres; one or more relay lens for transporting image to outside may be applied, or an image sensor (i.e. a sensor capable of converting optical signals into electric signals).

An optical system capable of zooming, e.g. a camera, having two fluid lenses may consume relatively small amount of space in a lateral direction of the optical system, but may have a relatively low zoom factor. An optical system capable of zooming with two displaceable lenses may have a high zoom factor, but normally consumes considerable space in a lateral direction of the optical system. Thus, the optical image probe according to the present invention with a displaceable image collector or image sensor and a fluid lens may have both a high zoom factor and is compact in a lateral direction.

Another advantage is to avoid the difficulty of scaling down a system with movable lenses (for small-diameter endoscopes). Such a system contains two small motors and several thin sliding rods. Small motors are difficult and expensive to make and the system is difficult to assemble. In our invention only one small motor is needed. Moreover, this motor can be somewhat larger than lens displacement motors, as there is more space behind the image collector than next to the lenses.

It may be mentioned that Japanese patent application JP 2006271503 discloses an endoscopic optical system where errors in quantization due to the limited resolution of the image sensor are reduced *inter alia* by displacing the image sensor while keeping the imaging lens fixed. However, this optical endoscope is only meant for focusing, not zooming. It is also not apparent that these measures may lead to a high zoom factor as obtained by the present invention. Thus, the endoscopic optical system disclosed in JP 2006271503 is not particularly relevant for the present invention.

In one embodiment, the fluid lens may be optically arranged for imaging a region of interest in front of the probe. Alternatively or additionally, the fluid lens may be optically arranged for imaging a region of interest next to the probe.

Beneficially, the probe may have a range of optical powers of the fluid lens defined by a maximum optical power (globally or locally) and a minimum optical power (globally or locally), the probe being optically arranged so that a corresponding range of focusing positions of the displaceable image collector enables focusing of the probe over a substantial part of the said range of optical powers. Thus, the fluid lens optical power range and focusing range of the image collector range may match each other. In this context a "substantial part" may mean at least 70%, 80% or 90%. The term "enable focusing" means in focus for the given purpose and application. Whether the image is "in focus" may conventionally be determined e.g. by edge detection, by high frequency analysis of the Fourier transformed (FT) signal, or other focusing techniques available or useable.

When zooming with a lens system having two fluid lenses and no moveable lenses, the fluid lenses must both change the focal length of the lens system as well as keeping the image in focus on the image sensor. In general the requirement of keeping the image in focus on the image sensor requires more optical power change than is required for the focal length change. This focusing requirement therefore limits the maximum achievable zoom factor. When, however, this focusing is performed by moving the image sensor, the above restriction is removed and a larger zoom factor becomes possible.

More specifically, the probe may have an optical power of the fluid lens and a corresponding focusing position of the image collector with an effective zoom factor of at least 2, preferably at least 2.5, or more preferably at least 3.0. The term "zoom factor" is understood to be a ratio of change in focal length. Other minimum value may be 1.6, or 1.8, or, alternatively, 3.5., 4.0.

Alternatively, the probe with two positions for the optical power of the fluid lens and two corresponding focusing positions of the image collector may have an effective zoom factor in the interval 1 to 4, preferably in the interval 1.5 to 3, or more preferably in the interval 1.5 to 2.5. New designs or liquid combinations may give much higher zoom factors. Thus, the intervals may alternatively be 1-5, 1-6, 2-5, or 2-6.

Typically, the fluid lens may consist of at least two immiscible fluids that are separated over a meniscus. In one embodiment, the shape of the meniscus in the fluid lens is changeable by pumping fluid into or out of the fluid container to provide a design that is easy to implement and avoids electrical wiring in the probe. In another embodiment, the fluid lens may be an electrowetting lens comprising two immiscible fluids. More particularly, the electrowetting lens may have an asymmetric electrode configuration so as to enable tilting of the meniscus formed between the two immiscible fluids so that the optical probe may repositioned the region of imaging by manipulating the meniscus.

The actuator may be an electromagnetic actuator, the electromagnetic actuator comprises an actuator housing, a driven member movably disposed with to the housing, preloading means for providing a normal force between the actuator housing and the driven member such that a friction force between the actuator housing and the driven member resulting from the normal force must be overcome to initiate a relative movement between the housing and the driven member, driving means for overcoming the said friction force and driving the driven member relative to the actuator housing. This actuator has the advantage that energy is only required during displacements not during fixed positions. Further details on this so-called friction stepper may be found WO2006/117715.

Alternatively, the actuator for displacing the image collector may be a piezoelectric actuator. In another embodiment, the said actuator may be a pneumatic or a hydraulic actuator, i.e. an actuator that converts fluid pressure into a corresponding displacement, e.g. a bellows. This has the particular advantage that electrical wiring and the use of electromagnets is avoided. Electrical wires can be negatively affected when heated for example during autoclave of an optical probe used for in-vivo inspection. Similarly magnets

Preferably, the probe may form part of an endoscope, a catheter, a needle, or a biopsy needle for in-vivo applications, though the present invention is not limited to these applications.

In a second aspect, the present invention relates to an optical imaging system, the system comprising:

- a light source (LS),
- a sample arm optically coupled to the light source, the sample arm having at its distal end an optical image probe according to the first aspect of the invention, and
- an imaging display device (IDD) coupled (C) to the light source (LS) and the sample arm.

In a third aspect, the present invention relates to a method for performing optical imaging, the method comprising

- providing a housing,
- providing a fluid lens positioned at an end portion of the housing, the fluid lens having a changeable optical power, and
- positioning an image collector within the housing, the image collector being arranged on an optical path of the fluid lens, the image collector being displaceable along the said optical path by an actuator.

The first, second, and third aspect of the present invention may each be combined with any of the other aspects. These and other aspects of the invention will be apparent from and elucidated with reference to the embodiments described hereinafter.

BRIEF DESCRIPTION OF THE FIGURES

The present invention will now be explained, by way of example only, with reference to the accompanying figures, where

Figure 1 is a schematic cross-sectional drawing of an optical image probe according to the present invention,
Figure 2 is another schematic cross-sectional drawing of an optical image probe according to the present invention,
Figure 3 is a schematic drawing of an optical imaging system according to the present invention,
Figure 4 shows simulations of the optical path through an optical image probe according to the present invention,
Figure 5 is drawing showing how the focal point is changeable by the fluid lens,
Figure 6 is a schematic cross-sectional drawing of a fluid lens in an optical image probe according to the present invention,
Figure 7 is a cross-sectional drawing of an electro-magnetic actuator for displacing an image sensor according to the present invention,
Figure 8 is a cross-sectional drawing of an piezoelectric actuator for displacing an image sensor according to the present invention,
Figure 9 is a cross-sectional drawing of a pressure-driven actuator for displacing an image sensor according to the present invention,
Figure 10 is a flow-chart of a method according to the invention, and
Figure 11 is a schematic view of a zoom lens made of two lenses.

DETAILED DESCRIPTION OF AN EMBODIMENT

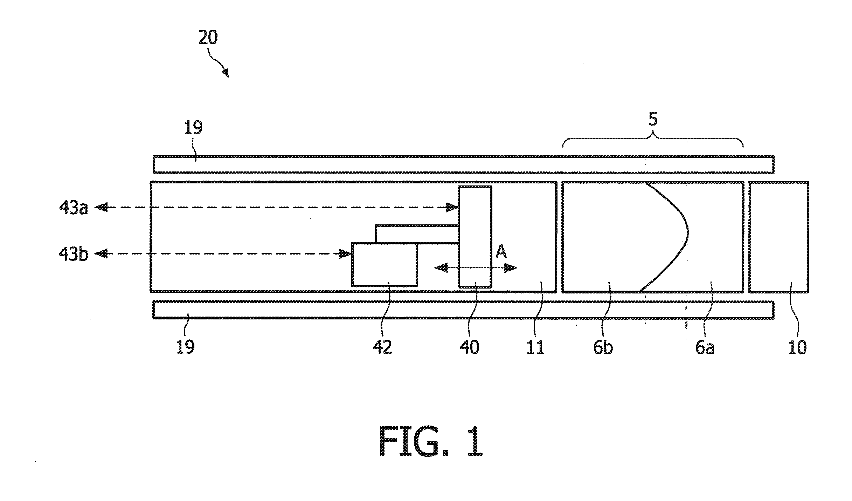

Figure 1 is a schematic cross-sectional drawing of an optical image probe 20 according to the present invention. The image probe 20 comprises a housing 19 surrounding and protecting the interior part of the probe. A fluid lens 5 is positioned at an end portion of the housing 19. The fluid lens 5 has a changeable optical power i.e. the focal depth of the lens can be changed on demand. The fluid lens 5 comprises at least two immiscible fluids 6a and 6b that are separated over an interfacing meniscus. A switchable lens can be made according to the principles described in US patent 7,126,903 B2 (Variable Focus Lens). In this reference, it is described how a variable-focus lens can be made with the smallest possible outer diameter. Such a small-diameter lens is obtained by placing two immiscible liquids in a short tube with an upper hydrophobic coating and an insulating layer. The two liquids have different refractive indices and therefore the meniscus between them forms a lens. By altering the curvature of the meniscus, the optical power of the lens can be varied. The container may also be non-cylindrical, e.g. conical as described in International patent application WO 00/58763, where further details regarding various shapes, and to how make these shapes may be found.

In front of the fluid lens 5, a further lens 10 is positioned. The lens 10 can also be replaced a window with no optical power, but normally the fluid lens works in cooperation with additional optics.

An image sensor 40 is positioned within the housing, the sensor being arranged on the optical path of the fluid lens i.e. behind the fluid lens 5 so that light captured by the fluid lens 5 and the lens 10 can be detected by the image sensor 40. The image sensor 40 is displaceably arranged along the optical path by an actuator 42. Thus, as indicated in Figure 1 by the arrow A, the image sensor 40 can be moved a certain range within the probe 20 so as to facilitate sufficient focusing of the region or object (not shown in Figure 1) to be imaged outside the probe 20.

As also indicated in Figure 1 by dashed arrows 43a, the image sensor 40, e.g. a charged coupled device (CCD) or a complementary metal oxide semi-conductor (CMOS) or other image sensors readily available to the skilled person, is connected to the exterior of the probe, i.e. for powering, monitoring, control, output, etc. It should be mentioned that the image sensor could also be one or more fibres displaceably arranged behind the fluid lens 5, the one or more fibres being optically arranged for transmitting the image to a corresponding image sensor. However, for the reminder of this description the image sensor, and displacement thereof, is discussed, but it will be apparent for the skilled reader that equivalently one or more fibres; one or more relay lens for transporting image to outside may be applied as the invention relates generally to displacement of an image collector.

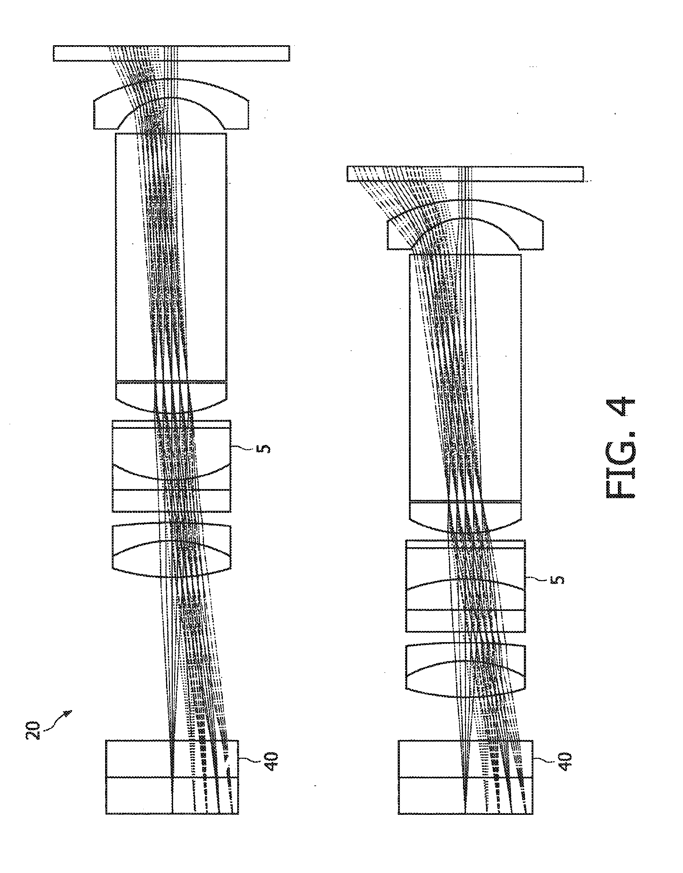

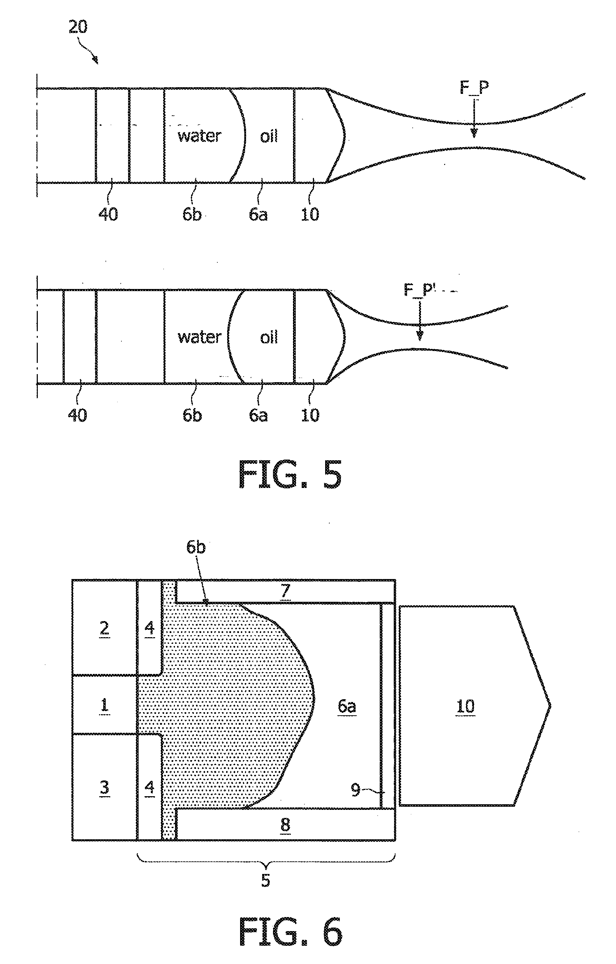

Region 11 denotes an area between the image sensor 40 and the fluid lens 5. Region 1 could be empty but several lens and other optical components could be positioned there as well. In Figure 4, a lens is positioned in region 11. In Figure 6, an optical fibre 1 is present in region 11.

Similarly, the actuator 43 is connected outside of the probe 20 for powering and control as indicated by the arrow 43b. In Figure 1, the image sensor 40 is connected to the actuator 42 via a rod, the rod being displaceable by the actuator 42, but other actuator configurations are of course readily available to the skilled person once the general principle of the present invention has been acknowledged.

In Figure 1, the probe 20, and particularly the fluid lens 5, is optically arranged for imaging a region of interest in front of the probe.

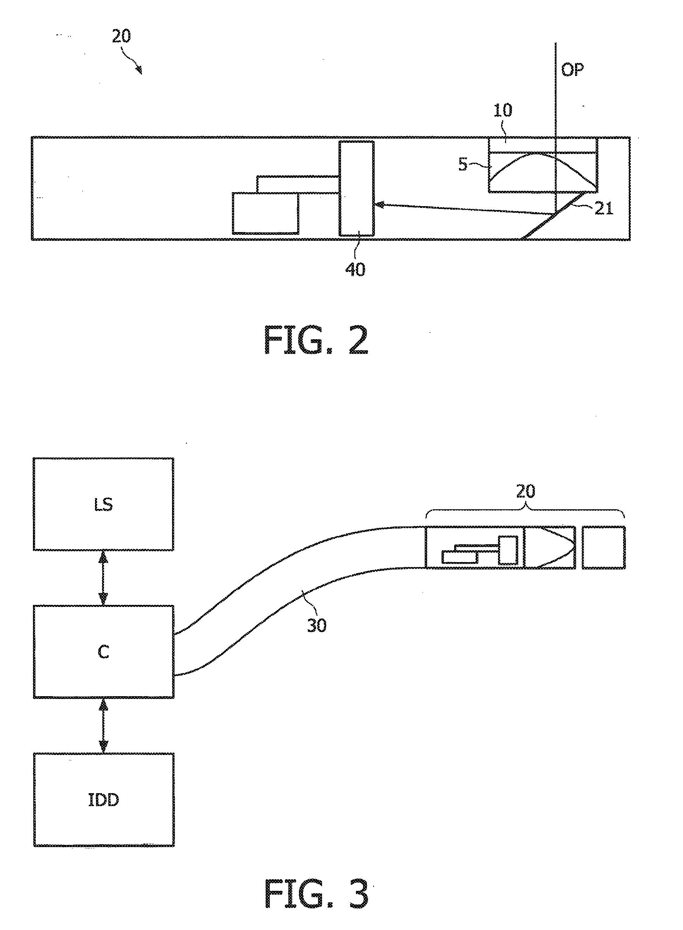

Figure 2 is another schematic cross-sectional drawing of an optical image probe 20 according to the present invention. Figure 2 is similar to Figure 1, but differs in that the fluid lens 5 is optically arranged for imaging a region of interest next to the probe 20. Certain elements are left out for clarity, e.g. the housing 19. Thus, light enters the side of the probe along the optical path OP. Together with mirror 21, the probe 20 can detect light on the image sensor 40. It is contemplated that a combination of front detection (i.e. Figure 1) and side detection (like Figure 2) is possible. Possibly, fluid lenses having a curvature may be applied, e.g. in a corner section of an optical probe 20 according to the present invention.

Figure 3 is a schematic drawing of an optical imaging system according to the present invention. The system comprises a light source LS, which may be positioned either at a rear position, as indicated in Figure 3, or embedded in or near the optical probe 20 itself. A sample arm 30 can be optically coupled to the light source LS and the light or, more generally, the illuminating radiation, can by appropriate optical conduction be lead to the probe 20 via the sample arm 30. The sample arm 30 has at its distal end an optical image probe 20 according to the present invention. At the rear of the probe 20, an imaging display device IDD for processing and displaying the image can be coupled via coupling unit C with the light source LS and the sample arm 30.

Figure 4 shows simulations of the optical path through an optical image probe 20 according to the present invention. To realize zooming in endoscopy with mechanical means around the lenses is difficult due to the limited space. A zoom lens based on fluid lens has the benefit of no additional mechanical parts around the lenses. This has the drawback that only a certain zoom factor can be reached due to the limited diopter change that can be reached by the fluid lens. In order to still be able to zoom one can make use of a single fluid lens 5 to change the focal length of the lens system (i.e. to zoom) and to use the displacement of the image sensor 40 to bring the system in focus. In figure 4 a design is shown, where the zoom factor is 1.6x, image sensor diagonal diameter 3.264mm, focal length in so-called tele configuration 3.46mm (upper of Figure 4) and in the wide configuration (lower of Figure 4) 2.09mm. The stop diameter is 0.88mm. In this design the image sensor 40 has to be displaced by 3.3mm. The switching range of the liquid lens required for this zoom factor of 1.6 is smaller than its maximum range. Higher zoom factors are possible if the full range is used. Models simulation of the modulation transfer studies shows satisfactory results, i.e. a relatively high transfer over a broad range of frequencies.

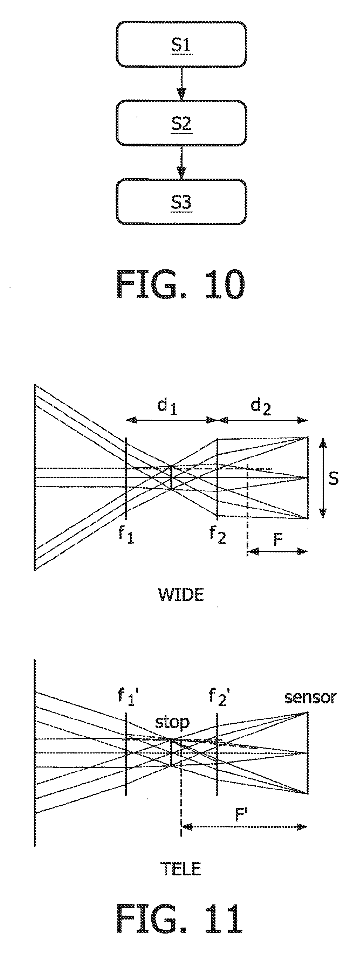

To demonstrate that larger zoom factors can be reached by a system consisting of a fluid lens and a moveable sensor compared to a system consisting of two fluid lenses, we consider the zoom lens system shown in Figure 11. It consists of a system of two lenses having a focal length of f1 and f2 in the WIDE zoom configuration and f1' and f2' in the TELE zoom configuration. The two lenses are separated by a distance d]. The distance between the second lens and the image sensor is d2. In the WIDE configuration the focal length of the total system is F and in the TELE configuration F'. Hence the zoom factor is given by F/F'. The size of the image sensor is S. The stop of the lens system is positioned between the two lenses. From paraxial calculation we can derive that the focal lengths of the two lenses f1 and f2 are related to the focal length of the total system F by

$$\text{WIDE: } f_1 = \frac{d_1}{1 - \dfrac{d_2}{F}} \quad \text{and} \quad f_2 = \frac{d_1 d_2}{d_1 + d_2 - F} \; , \tag{1}$$

$$\text{TELE: } f_1' = \frac{d_1}{1 - \dfrac{d_2}{F'}} \quad \text{and} \quad f_2' = \frac{d_1 d_2}{d_1 + d_2 - F'} \; . \tag{2}$$

Consider as an example the case d1 = 5mm and d2 = 5mm. Let the switching range of the fluid lenses be limited by |f1| > 6mm and |f2| > 6mm. In this case the focal range change possible is 5.83mm < F < 14.17mm, resulting in a zoom factor of 2.43. This range is limited by the constraint on f2. When we consider the case where the second lens is replaced by a fixed lens with f2 = -2mm and an image sensor that can be displaced between 2mm < d2 < 20mm, we find that the focal length F can change between 12.0mm < F < 75.0 mm, showing a zoom factor of 6.25.

Figure 5 is a drawing showing how the focal point is changeable by the fluid lens 5, thus

Figure 5 shows two schematic cross-sectional views of an optical image probe 20 according to the present invention having two different settings of the optical power for the fluid lens 5. The lens 5 could comprise a water phase 6b to the left and an oil phase 6a to the right.

In the top view, the meniscus of the fluid interface is curved towards the oil phase resulting in a focal point F_P as indicated in the top view of Figure 5. Similarly, in the bottom view, the meniscus of the fluid interface is curved towards the water phase resulting in another focal point F_P' as indicated in the bottom view of Figure 5, the focal point thereby being shifted towards the lens 10 and also the image sensor 40 is displaced further away from the lens 5 so as to allow for focusing. Thus, application of the optical image probe 20 facilitates manipulation of the focal point F_P, in particular so as to enable imaging with a sufficient focusing by displacement of the image sensor 40.

Figure 6 is a schematic cross-sectional drawing of a fluid lens 5 in an optical image probe 20 according to the present invention. Figure 6 is a schematic cross-sectional view of an optical image probe with asymmetric electrode configuration according to the present invention where annotation 1 marks a fiber connection to the fluid lens i.e. fiber 1 is positioned in the region 11. In order to minimize the fiber-outcoupling losses, one has to make sure that the refractive index of the first fluid 6b and that of the core of the fiber 1 are as close as possible. When the end part of the fiber (specifically the cladding) is *n*-type doped such that is becomes electrically active (e.g. by incorporation sufficient quantities of arsenic, phosphorous or another group V element), the electrode 4 may be removed. In this case, contacting of the bottom electrode 4 can occur outside of the fiber.

The second liquid 6a should have a different refractive index as compared to the first liquid 6b in order to allow for lens-action when the meniscus between the two liquids is curved. The two liquids are required to be immiscible to obtain a stable meniscus. By suitable choice of the surface tension between the two liquids and the geometry of the cavity (cylindrical or conical), both concave and convex lens action can be obtained.

The electrodes on the sides as indicated by 7 and 8 could be a series of electrodes i.e. more than the two electrodes drawn for simplicity here, that are equidistantly spaced on the circumference of the lens. In order to prevent short-circuits, the side electrodes do not run all the way to the bottom electrode 4 as indicated by the black liquid between electrode 4 and electrode 7 and 8, respectively. By applying the same voltage on all electrodes, a spherical meniscus can be obtained as a special case. However, by suitable voltage differences between these electrodes, the plane of the meniscus can be tilted as desired. This will result in a directionality of the focal point of the lens, allowing e.g. a surgeon to focus on objects that are not directly in front of the lens system. Such a tilted meniscus will also enable a surgeon to illuminate around a corner during an in vivo inspection. Due to the finite number of electrodes placed around the two fluids 6a and 6b, no perfectly tilted meniscus will be possible. Therefore, aberrations and wave front errors will be introduced to some extent.

Finally, an optically transparent cover layer 9 is placed on top of the lens in order to prevent liquid leaking out of the liquid lens 5. Adjacent to the cover layer 9, the high NA lens 10 is positioned. Possibly, layer 9 and lens 10 may be combined into one and the same entity.

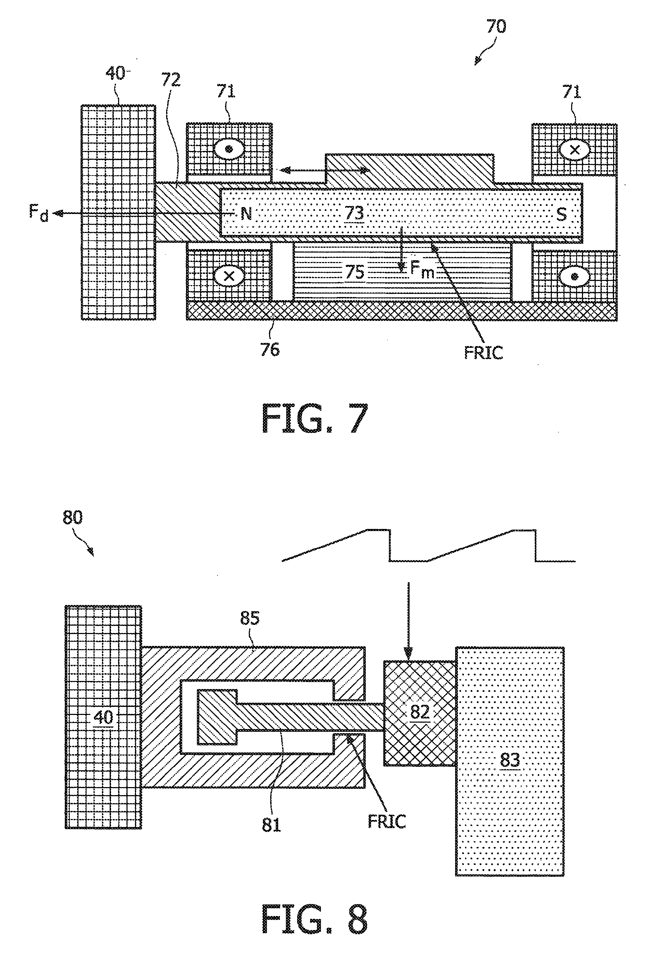

Figure 7 is a cross-sectional drawing of an electro-magnetic actuator 70 for displacing an image sensor according to the present invention. Thus, actuator 70 is a specific actuator of the more general actuator 42 shown in Figures 1 and 2. The actuator which needs to move image sensor 40 should be small enough to fit in an endoscope and strong enough to displace image sensor 40. Furthermore, self-braking characteristics of such an actuator would be very useful, since no energy is needed for maintaining the image sensor 40 at the wanted focus position.

One such actuator is an electro-magnetic actuator, a so-called friction stepper. This actuator 70 with an image sensor 40 is shown in figure 7. The actuator 70 consists of a magnet which is placed in two coils 71. In order to connect the image sensor 40 to the magnet 73, the magnet 73, magnet holder 72 and the sensor 40 can be fixed to each other as shown in Figure 7. The holder 72 should be made of a material with a relative magnetic permeability close to 1 (e.g. plastic). Furthermore, no contact exists between magnet holder 72, magnet 73, image sensor 40 at one side and coils 71 at another side. Magnet support 75, coils 71 and ferric member 76 are fixed to each other, see Figure 7.

When no current $I$ runs through the coils, the magnet 73 stands still with respect to the coils 71 and steel plate 76 due to the magnetic attraction force $F_m$ between magnet 73 and the ferric member 76, and due to a friction force FRIC between the magnet holder 72 and the magnet support 75. Next, if current runs through the coil 71, a driving force $F_d$ occurs and the image sensor 40 can move to the right or to the left direction, depending on the current through the coils 71. To move the image sensor 40 in small steps, current pulses should be applied to the coils 71.

In order to check the feasibility of such a solution, Opera 3D simulation of the magnetic field has been performed. It is assumed that the actuator 40 can be placed into a tube with 3mm inner diameter and the length of the actuator is 10mm without the image sensor. Furthermore, the mass of the moving part of the actuator (image sensor 40, magnet 73 and magnet holder 72) is 0.25g. In order to have no movement of the moving part due to 3G shock, the attractive force $F_m$ should be greater than 0.036N and the driving force $F_d$ should be higher than 0.01N. According to Opera 3D simulation, the attractive force of the simulated actuator is $F_m \in [0.046N, 0.051N]$ and $F_d \in [0.080N, 0.087N]$. Consequently, the simulated actuator 70 can be used for moving the image sensor 40.

Further details on the so-called friction stepper can be found in International patent application WO 2006/117715, where an actuator including: a housing; a driven member movably disposed with respect to the housing; a magnet associated with the driven member and a ferric member for providing a magnetic attraction force between the driven member and the ferric member for providing a normal force, Fn, between the housing and driven member such that a friction force between the housing and driven member resulting from the normal force must be overcome to initiate a relative movement between the housing and driven member; and a magnetic drive system for overcoming the friction force and driving the driven member relative to the housing. Preloading means may comprise a magnet associated with the driven member and ferric member for providing a magnetic attraction force between the driven member and the ferric member, wherein the magnetic attraction force is substantially equal to the normal force.

The electro-magnetic actuator 80 can beneficially be implemented in combination with a displacement position as disclosed in US patent 5399952. The actuator i.e. the electromagnetic drive system includes a motor having a first motor section movable relative to a second motor section along a motion axis. The second motor section has three excitation coils. The first motor section includes a magnetic circuit generating a magnetic field in the excitation coils. The first motor section also includes a short-circuit winding for only high-frequency induction currents. This short-circuit winding is magnetically coupled to the excitation coils. The degree of electromagnetic coupling of at least one excitation coil depends on the position of the first motor section relative to the second motor section. The short-circuit winding provides a position-dependent electromagnetic coupling between the center excitation coil and the two outer excitation coils. The position-dependent coupling can be detected by superimposing a high-frequency detection signal on the excitation system for the center coil and by detecting currents induced in the two outer coils by the high-frequency detection signal.

Figure 8 is a cross-sectional drawing of an piezoelectric actuator 80 for displacing an image sensor 40. The actuator 80 comprises a piezoelectric element 82 and a driving shaft 81. The piezoelectric element 82 is at one side (In Figure 8 the right end) connected to a fixed position (i.e. actuator support 83 in Figure 8) and at the other side to the driving shaft 81. At the right end of the driving shaft 81, a moving element is fixed (clamped) to the shaft via a friction force FRIC and the image sensor 40 is fixed to the moving element. In order for the moving part with the image sensor 40 to make a step, a waveform, as shown in Figure 8 upper right part, could be applied to the piezoelectric element as indicated by the solid arrow. The step signal consists of a slowly increasing and a fastly decreasing voltage or of a fastly increasing and a slowly decreasing voltage. The moving part makes a step with respect to the fixed position 83 during the slowly decreasing (increasing) part of the signal, and it makes a step with respect to the driving shaft 81 during the fastly decreasing (increasing) part of the signal. However, during fast change of the signal, the moving part practically does not move with respect to the fixed coordinate frame due to inertia of the moving part.

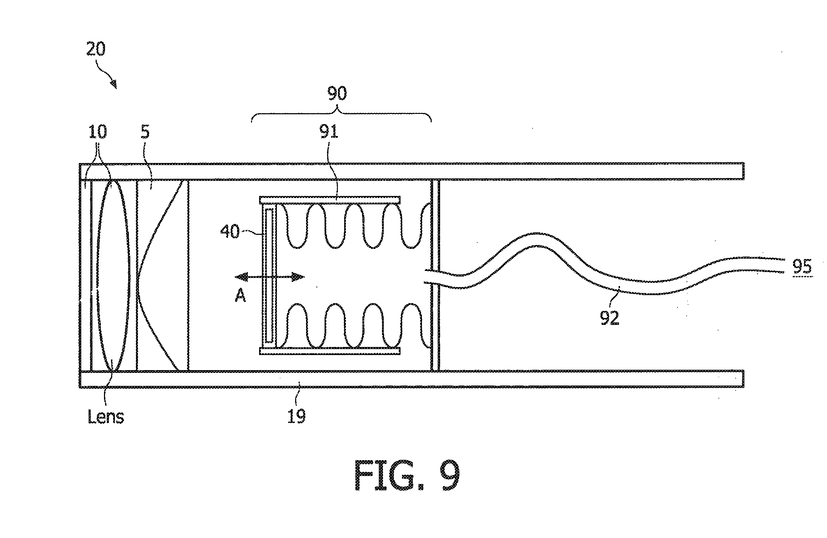

Figure 9 is a cross-sectional drawing of a pressure-driven actuator 90 for displacing an image sensor 40 according to the present invention. A bellows is a hollow cylinder with a spring-shaped wall. It can change its length under influence of a fluid pressure i.e. a gas or a liquid under pressure. The fluid pressure can be applied through a hollow tube 92. The pressure in the tube can be adapted on the backside of the probe or endoscope 20, hence outside the body when applied in-vivo. An increase in pressure will expand the bellows 90, resulting in a shift to the left as indicated by solid arrow A. A decrease in pressure will shift the image sensor 40 to the right. Additional rods or grooves can prevent sensor tilt. The sensor can slide along the rods or grooves. It is also possible to attach the sensor to a sliding cylinder 91 that fits around the bellows, or that fits inside the endoscope exterior wall 19. The pressure can be applied in various ways, e.g. by squeezing a flexible end part of the tube 92. This (electromechanical) squeezing element does not need to be miniaturized, as it is in the part of the endoscope outside of the body. Also, it is possible to connect the fluid inside the tube via a flexible membrane with an air pressurising device 95 outside the endoscope 20.

Figure 10 is a flow-chart of a method according to the invention. The method comprising

- **S1** providing a housing 19,
- **S2** providing a fluid lens 5 positioned at an end portion of the housing, the fluid lens having a changeable optical power, and
- **S3** positioning an image collector 40 within the housing, the image collector being arranged on an optical path of the fluid lens, the image collector being displaceable along the said optical path by an actuator 42, 70, 80 or 90.

The invention can be implemented in any suitable form including hardware, software, firmware or any combination of these. The invention or some features of the invention can be implemented as computer software running on one or more data processors and/or digital signal processors. The elements and components of an embodiment of the invention may be physically, functionally and logically implemented in any suitable way. Indeed, the functionality may be implemented in a single unit, in a plurality of units or as part of other functional units. As such, the invention may be implemented in a single unit, or may be physically and functionally distributed between different units and processors.

Although the present invention has been described in connection with the specified embodiments, it is not intended to be limited to the specific form set forth herein. Rather, the scope of the present invention is limited only by the accompanying claims. In the claims, the term "comprising" does not exclude the presence of other elements or steps. Additionally, although individual features may be included in different claims, these may possibly be advantageously combined, and the inclusion in different claims does not imply that a combination of features is not feasible and/or advantageous. In addition, singular references do not exclude a plurality. Thus, references to "a", "an", "first", "second" etc. do not preclude a plurality. Furthermore, reference signs in the claims shall not be construed as limiting the scope.

**Claims**

1.  An optical image probe (20) comprising

    - a housing (19),
    - a fluid lens (5) positioned at an end portion of the housing, the fluid lens having a changeable optical power, and
    - an image collector (40) positioned within the housing, the image collector being arranged on an optical path of the fluid lens, **characterised in that** the image collector being displaceable along the said optical path by an actuator (42, 70, 80, 90).

2.  The probe according to claim 1, wherein the fluid lens (5) is optically arranged for imaging a region of interest in front of the probe (20).

3.  The probe according to claim 1, wherein the fluid lens (5) is optically arranged for imaging a region of interest next to the probe (20).

4.  The probe according to claim 1, wherein the probe (20) has a range of optical powers of the fluid lens (5) defined by a maximum optical power and a minimum optical power, the probe being optically arranged so that a corresponding range of focusing positions of the displaceable image collector (40) enables focusing of the probe over a substantial part of the said range of optical powers.

5.  The probe according to claim 1 or claim 4, wherein the probe (20) with an optical power of the fluid lens (5) and a corresponding focusing position of the image collector (40) has an effective zoom factor of at least 2, preferably at least 2.5, or more preferably at least 3.0.

6.  The probe according to claim 1 or claim 4, wherein the probe with two positions for the optical power of the fluid lens and two corresponding focusing positions of the image collector has an effective zoom factor in the interval 1 to 4, preferably in the interval 1.5 to 3, or more preferably in the interval 1.5 to 2.5.

7.  The probe according to claim 1, wherein the fluid lens (5) consist of at least two immiscible fluids (6a, 6b) that are separated over a meniscus.

8.  The probe according to claim 7, wherein the shape of the meniscus in the fluid lens (5) is changeable by pumping fluid into or out of a fluid container holding the fluid.

9. The probe according to claim 7, wherein the fluid lens (5) is an electrowetting lens comprising two immiscible fluids (6a, 6b).

10. The probe according to claim 9, wherein the electrowetting lens (5) has an asymmetric electrode configuration (7, 8) so as to enable tilting of the meniscus formed between the two immiscible fluids (6a, 6b).

11. The probe according to claim 1, wherein the said actuator is an electromagnetic actuator comprising:

   - an actuator housing (19),
   - a driven member (72, 73) movably disposed with respect to the housing,
   - preloading means (75, 76) for providing a normal force between the actuator housing and the driven member such that a friction force (FRIC) between the actuator housing and the driven member resulting from the normal force must be overcome to initiate a relative movement between the housing and the driven member,
   - driving means (71) for overcoming the said friction force and driving the driven member relative to the actuator housing.

12. The probe according to claim 1, wherein the said actuator is a piezoelectric actuator (80) or a pneumatic actuator a hydraulic actuator (90).

13. The probe according to claim 1, wherein the probe forms part of an endoscope, a catheter, a needle, or a biopsy needle.

14. An optical imaging system, the system comprising:

   - a light source (LS),
   - a sample arm (30) optically coupled to the light source, the sample arm having at its distal end an optical image probe (20) according to claim 1, and
   - an imaging display device (IDD) coupled (C) to the light source (LS) and the sample arm (30).

15. A method for performing optical imaging, the method comprising

   - providing a housing (19),
   - providing a fluid lens (5) positioned at an end portion of the housing, the fluid lens having a changeable optical power, and
   - positioning an image collector (40) within the housing, the image collector being arranged on an optical path of the fluid lens, **characterised in that** the image collector being displaceable along the said optical path by an actuator (42, 70, 80, 90).

**Patentansprüche**

1. Optische Bildsonde (20), die Folgendes umfasst:

   - ein Gehäuse (19),
   - eine Flüssigkeitslinse (5), die an einem Endteil des Gehäuses angeordnet ist, wobei die Flüssigkeitslinse eine veränderbare optische Leistung hat, und
   - einen Bildkollektor (40), der innerhalb des Gehäuses angeordnet ist, wobei der Bildkollektor auf einem optischen Pfad der Flüssigkeitslinse vorgesehen ist, **dadurch gekennzeichnet, dass** der Bildkollektor durch ein Betätigungselement (42, 70, 80, 90) entlang des genannten optischen Pfades verschiebbar ist.

2. Sonde nach Anspruch 1, wobei die Flüssigkeitslinse (5) optisch zum Abbilden einer vor der Sonde (20) befindlichen interessierenden Region ausgelegt ist.

3. Sonde nach Anspruch 1, wobei die Flüssigkeitslinse (5) optisch zum Abbilden einer neben der Sonde (20) befindlichen interessierenden Region ausgelegt ist.

4. Sonde nach Anspruch 1, wobei die Sonde (20) einen Bereich an optischen Leistungen der Flüssigkeitslinse (5) hat, der durch eine maximale optische Leistung und eine minimale optische Leistung definiert ist, wobei die Sonde optisch derartig ausgelegt ist, dass ein entsprechender Bereich an Fokussierpositionen des verschiebbaren Bildkollektors (40) das Fokussieren der Sonde über einen wesentlichen Teil des genannten Bereichs an optischen Leistungen ermöglicht.

5. Sonde nach Anspruch 1 oder Anspruch 4, wobei die Sonde (20) mit einer optischen Leistung der Flüssigkeitslinse (5) und einer entsprechenden Fokussierposition des Bildkollektors (40) einen effektiven Zoomfaktor von mindestens 2, vorzugsweise mindestens 2,5, oder noch mehr zu bevorzugen von mindestens 3,0 hat.

6. Sonde nach Anspruch 1 oder Anspruch 4, wobei die Sonde mit zwei Positionen für die optische Leistung der Flüssigkeitslinse und zwei entsprechenden Fokussierpositionen des Bildkollektors einen effektiven Zoomfaktor im Intervall 1 bis 4 hat, vorzugsweise im Intervall 1,5 bis 3, oder noch mehr zu bevorzugen im Intervall 1,5 bis 2,5.

7. Sonde nach Anspruch 1, wobei die Flüssigkeitslinse (5) aus mindestens zwei unvermischbaren Flüssigkeiten (6a, 6b) besteht, die über einen Meniskus getrennt sind.

8. Sonde nach Anspruch 7, wobei die Form des Meniskus in der Flüssigkeitslinse (5) veränderbar ist, indem Flüssigkeit in einen die Flüssigkeit enthaltenden Flüssigkeitsbehälter hineingepumpt oder daraus herausgepumpt wird.

9. Sonde nach Anspruch 7, wobei die Flüssigkeitslinse (5) eine elektrobenetzende Linse (5) mit zwei unvermischbaren Flüssigkeiten (6a, 6b) ist.

10. Sonde nach Anspruch 9, wobei die elektrobenetzende Linse (5) eine asymmetrische Elektrodenkonfiguration (7, 8) hat, um das Neigen des zwischen den beiden unvermischbaren Flüssigkeiten (6a, 6b) gebildeten Meniskus zu ermöglichen.

11. Sonde nach Anspruch 1, wobei das genannte Betätigungselement ein elektromagnetisches Betätigungselement ist, das Folgendes umfasst:

    - ein Betätigungselement-Gehäuse (19),
    - ein angetriebenes Element (72, 73), das in Bezug auf das Gehäuse beweglich angeordnet ist,
    - Vorbelastungsmittel (75, 76) zum Bereitstellen einer Normalkraft zwischen dem Betätigungselement-Gehäuse und dem angetriebenen Element, so dass die Reibungskraft (FRIC) zwischen dem Betätigungselement-Gehäuse und dem angetriebenen Element, die aus der Normalkraft resultiert, überwunden werden muss, um eine relative Bewegung zwischen dem Gehäuse und dem angetriebenen Element zu initiieren,
    - Antriebsmittel (71) zum Überwinden der genannten Reibungskraft und zum Antreiben des angetriebenen Elements in Bezug auf das Betätigungselement-Gehäuse.

12. Sonde nach Anspruch 1, wobei das genannte Bestätigungselement ein piezoelektrisches Betätigungselement (80) oder ein pneumatisches Betätigungselement eines hydraulischen Betätigungselements (90) ist.

13. Sonde nach Anspruch 1, wobei die Sonde einen Teil eines Endoskops, eines Katheters, einer Kanüle oder einer Biopsienadel bildet.

14. Optisches Bildgebungssystem, wobei das System Folgendes umfasst:

    - eine Lichtquelle (LS),
    - einen Probenarm (30), der optisch mit der Lichtquelle gekoppelt ist, wobei der Probenarm an seinem distalen Ende eine optische Bildsonde (20) nach Anspruch 1 hat, und
    - eine Bildgebungsanzeigevorrichtung (IDD), die mit der Lichtquelle (LS) und dem Probenarm (30) gekoppelt (C) ist.

15. Verfahren zum Durchführen einer optischen Bildgebung, wobei das Verfahren Folgendes umfasst:

    - Bereitstellen eines Gehäuses (19),
    - Bereitstellen einer Flüssigkeitslinse (5), die an einen Endteil des Gehäuses positioniert ist, wobei die Flüssigkeitslinse eine veränderbare optische Leistung hat, und
    - Positionieren eines Bildkollektors (40) innerhalb des Gehäuses, wobei der Bildkollektor auf einem optischen Pfad der Flüssigkeitslinse angeordnet ist, **dadurch gekennzeichnet, dass** der Bildkollektor durch ein Betätigungselement (42, 70, 80, 90) entlang des genannten optischen Pfads verschiebbar ist.

**Revendications**

1. Sonde d'image optique (20) comprenant

   - un logement (19),
   - une lentille fluide (5) positionnée à une portion d'extrémité du logement, la lentille fluide ayant une puissance optique changeable, et
   - un collecteur d'images (40) positionné à l'intérieur du logement, le collecteur d'images étant agencé sur un chemin optique de la lentille fluide, **caractérisé en ce que** le collecteur d'images peut être déplacé le long dudit chemin optique par un actionneur (42, 70, 80, 90).

2. Sonde selon la revendication 1, dans laquelle la lentille fluide (5) est agencée optiquement pour imager une région d'intérêt à l'avant de la sonde (20).

3. Sonde selon la revendication 1, dans laquelle la lentille fluide (5) est agencée optiquement pour imager une région d'intérêt à côté de la sonde (20).

4. Sonde selon la revendication 1, dans laquelle la sonde (20) a une plage de puissances optiques de la lentille fluide (5) définie par une puissance optique maximale et une puissance optique minimale, la sonde étant agencée optiquement de sorte qu'une plage correspondante de positions de focalisation du collecteur d'images déplaçable (40) permette la focalisation de la sonde sur une partie substantielle de ladite plage de puissances optiques.

5. Sonde selon la revendication 1 ou 4, dans laquelle la sonde (20) avec une puissance optique de la lentille fluide (5) et une position de focalisation correspondante du collecteur d'images (40) a un facteur de zoom efficace d'au moins 2, de préférence d'au moins 2,5 ou avec plus de préférence d'au moins 3,0.

6. Sonde selon la revendication 1 ou 4, dans laquelle la sonde avec deux positions pour la puissance optique de la lentille fluide et deux positions de focalisation correspondantes du collecteur d'images a un facteur de zoom efficace dans l'intervalle de 1 à 4, de préférence dans l'intervalle de 1,5 à 3, ou avec plus de préférence dans l'intervalle de 1,5 à 2,5.

7. Sonde selon la revendication 1, dans laquelle la lentille fluide (5) se compose d'au moins deux fluides immiscibles (6a, 6b) qui sont séparés sur un ménisque.

8. Sonde selon la revendication 7, dans laquelle la forme du ménisque dans la lentille fluide (5) est changeable en pompant un fluide dans un contenant de fluide qui contient le fluide ou hors de celui-ci.

9. Sonde selon la revendication 7, dans laquelle la lentille fluide (5) est une lentille d'électromouillage comprenant deux fluides immiscibles (6a, 6b).

10. Sonde selon la revendication 9, dans laquelle la lentille d'électromouillage (5) a une configuration d'électrodes asymétriques (7, 8) afin de permettre une inclinaison du ménisque formé entre les deux fluides immiscibles (6a, 6b).

11. Sonde selon la revendication 1, dans laquelle ledit actionneur est un actionneur électromagnétique comprenant :

    - un logement d'actionneur (19),
    - un organe entraîné (72, 73) disposé de manière à pouvoir se déplacer par rapport au logement,
    - un moyen de précharge (75, 76) pour fournir une force normale entre le logement d'actionneur et l'organe entraîné de sorte qu'une force de frottement (FRIC) entre le logement d'actionneur et l'organe entraîné découlant de la force normale puisse être surmontée pour engendrer un mouvement relatif entre le logement et l'organe entraîné,
    - un moyen d'entraînement (71) pour surmonter ladite force de frottement et entraîner l'organe entraîné par rapport au logement d'actionneur.

12. Sonde selon la revendication 1, dans laquelle ledit actionneur est un actionneur piézoélectrique (80), un actionneur pneumatique ou un actionneur hydraulique (90).

**13.** Sonde selon la revendication 1, dans laquelle la sonde fait partie d'un endoscope, d'un cathéter, d'une aiguille ou d'une aiguille de biopsie.

**14.** Système d'imagerie optique, le système comprenant

    - une source lumineuse (LS),
    - un bras d'échantillon (30) couplé optiquement à la source lumineuse, le bras d'échantillon comportant, à son extrémité distale, une sonde d'image optique (20) selon la revendication 1, et
    - un dispositif d'affichage d'imagerie (IDD) couplé (C) à la source lumineuse (LS) et au bras d'échantillon (30).

**15.** Procédé d'exécution d'imagerie optique, le procédé comprenant

    - la fourniture d'un logement (19),
    - la fourniture d'une lentille fluide (5) positionnée à une portion d'extrémité du logement, la lentille fluide ayant une puissance optique changeable, et
    - le positionnement d'un collecteur d'images (40) à l'intérieur du logement, le collecteur d'images étant agencé sur un chemin optique de la lentille fluide, **caractérisé en ce que** le collecteur d'images peut être déplacé le long dudit chemin optique par un actionneur (42, 70, 80, 90).

FIG. 1

FIG. 2

FIG. 3

FIG. 4

FIG. 5

FIG. 6

FIG. 7

FIG. 8

FIG. 9

# FIG. 10

WIDE

TELE

# FIG. 11

**EP 2 300 857 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7126903 B2
- US 20070156021 A
- US 4930861 A
- JP 2006271503 B
- WO 2006117715 A
- WO 0058763 A
- US 5399952 A